# EUROPÄISCHE PATENTANMELDUNG

(11) **EP 1 118 346 A2**
(43) Veröffentlichungstag der Anmeldung: **25.07.2001**
(21) Anmeldenummer: 01100038.7
(22) Anmeldetag: 08.01.2001
(51) Int. Cl.: A61M 16/06

(54) **Nasale Beatmungsmaske und Maskenkissen für eine nasale Beatmungsmaske**

(30) Priorität: 21.01.2000 DE 10002571; 21.01.2000 DE 20001040 U
(71) Anmelder: MPV-Truma Gesellschaft Für Medizintechnische Produkte mbH, 85640 Putzbrunn bei München (DE)
(72) Erfinder: Hecker, Karl-Heinz, 83229 Aschau (DE); Schinagl, Rudolf, 82008 Unterhaching (DE)
(74) Vertreter: Leske, Thomas, Dr.

(57) **Zusammenfassung**

Die Erfindung betrifft eine nasale Beatmungsmaske bzw. ein Maskenkissen für eine nasale Beatmungsmaske. Die nasale Beatmungsmaske umfaßt ein Maskenkissen und einen Maskenhalteteil, welcher mit einem Schlauchanschluß für einen Beatmungsschlauch in Verbindung steht, und mindestens ein Gurtband zur Positionierung der Beatmungsmaske auf dem Gesicht, insbesondere auf der Nase, eines Benutzers. Das Maskenkissen für die nasale Beatmungsmaske bildet mit dem Maskenteil einen Innenraum zur Aufnahme zumindest der Nase des Benutzers. Das Maskenkissen besteht aus flexiblem Material mit einer Dichtlippe, wobei die Dichtlippe bei Gebrauch der Beatmungsmaske mit einem Teil ihrer äußeren Oberfläche auf dem Gesicht des Benutzers abdichtend aufliegt und sich dabei verformt. Die Dichtlippe begrenzt dabei in dem aufliegenden Teil mit einer Kante eine Öffnung zum Innenraum und kann eine Verdickung aufweisen. Ferner kann das Maskenkissen eine inneren Stützwand und einer nach innen um die Stützwand geschlagenen Dichtlippe aufweisen, wobei die Dichtlippe auf der dem Innenraum abgewandten Seite der Stützwand verläuft und von dieser beabstandet ist, und wobei die Stützwand mit Stegen versehen ist, welche bei Gebrauch der Beatmungsmaske deren Verformung verringern.

## Beschreibung

Die Erfindung betrifft eine nasale Beatmungsmaske gemäß Anspruch 22 bzw. 23 und ein Maskenkissen für eine nasale Beatmungsmaske gemäß Anspruch 1 bzw. 12.

Nasale Beatmungsmasken dienen zur Versorgung mit Atemluft, beispielsweise zu medizinischen oder therapeutischen Zwecken.

Nasale Beatmungsmasken bestehen üblicherweise aus einem Maskenhalteteil mit zumindest einem Gurtband zum Positionieren der Beatmungsmaske auf dem Gesicht, insbesondere der Nase, eines Benutzers und einem teilelastischen, anatomisch geformten Maskenkissen, welches mit dem Maskenhalteteil lösbar verbunden sein kann. Die Beatmungsmaske wird somit gegen das Gesicht des Benutzers gedrückt. Über einen Schlauchanschluß am Maskenhalteteil kann die Beatmungsluft durch einen Beatmungsschlauch zugeführt werden. Die Beatmungsluft stammt dabei aus einem überdruckerzeugenden Gerät.

Die herkömmlich verwendeten Maskenkissen des Standes der Technik bestehen üblicherweise aus einem flexiblen Material. Der Bereich solcher Maskenkissen, welcher die nasale Beatmungsmaske abdichtend mit dem Gesicht eines Benutzers verbindet bzw. zumindest einen Teil des Gesichts eines Benutzers abdichtend umschließt, besteht dabei üblicherweise aus einem luft- oder fluidgefüllten Polster oder er besteht einfach aus einer wulstförmigen Ausformung des flexiblen Maskenkissenmaterials. Bei der Benutzung entsprechender Maskenkissen kann es jedoch zu undichten Stellen kommen, falls das Maskenkissen auf dem Gesicht des Benutzers, beispielsweise durch Krafteinwirkung aufgrund heftiger Kopfbewegungen des Benutzers, verrutscht. Üblicherweise wird dies dadurch verhindert bzw. eingeschränkt, daß die nasalen Beatmungsmasken, welche solche Maskenkissen verwenden, besonders fest durch entsprechende Gurtbänder auf das Gesicht bzw. die Gesichtskontur des Benutzers gepreßt werden. Dies vermindert entscheidend den Tragekomfort, welcher durch nasale Beatmungsmasken mit solchen Maskenkissen erzielbar ist.

Bei einem zweiten Maskenkissentyp, welcher bei nasalen Beatmungsmasken Verwendung findet, ist ein eingeschlagener Dichtabschnitt des Maskenkissens, welcher sehr flexibel ist, so angeordnet, daß er bei Benutzung einer nasalen Beatmungsmaske mit einem entsprechenden Maskenkissen durch den Druck des Beatmungsgases das Maskenkissen bzw. die nasale Beatmungsmaske gegen das Gesicht des Benutzers abdichtet. Zwar sind die Kräfte, welche bei dieser Art des Maskenkissens zur abdichtenden Positionierung und zum Haltern der gesamten nasalen Beatmungsmaske benötigt werden, deutlich geringer als im Fall eines Maskenkissens, welches weiter oben beschrieben wurde, jedoch besteht aufgrund der Tatsache, daß die genaue Anlage des sehr flexiblen Teils des Maskenkissens auf dem Gesicht eines Benutzers für die Abdichtung verantwortlich ist, die Anforderung, daß eine gute und zuverlässige Anlage dieses flexiblen Abschnitts des Maskenkissens an die Gesichtskontur des Benutzers erreicht werden muß. Dabei darf es insbesondere nicht zu Falten oder ähnlichem im entsprechenden Bereich des Maskenkissens kommen. Diese können aufgrund der sehr flexiblen Gestaltung entsprechender Maskenkissen schon beim Positionieren des Maskenkissens oder durch Bewegung des Benutzers und somit durch Verformung des Maskenkissens auftreten. Dies führt zu nicht optimalen Abdichteigenschaften solcher Maskenkissen.

Um einen bequemen Tragekomfort der Beatmungsmaske und eine einwandfreie Funktionsweise sicherzustellen, ist es notwendig das flexible Material des Maskenkissens möglichst dünnwandig auszubilden. Diese dünnwandig ausgeführten Maskenkissen bringen allerdings das Problem mit sich, nicht sehr haltbar zu sein, d. h. es kommt bei längerer Benutzung der Beatmungsmaske bzw. nach häufigem Auf- und Absetzen derselben zu Rißbildungen, insbesondere um die zur Aufnahme einer Nase dienende Öffnung des Maskenkissens herum, und die Dichtwirkung ist nicht länger gegeben. Ein häufiges Auswechseln bzw. Ersetzen des Maskenkissens einer solchen Beatmungsmaske verursacht erhebliche Kosten für den Benutzer.

Die Aufgabe der Erfindung besteht darin, die Nachteile des Standes der Technik zu überwinden und insbesondere ein Maskenkissen bzw. eine nasale Beatmungsmaske zu schaffen, welche sehr gute Abdichteigenschaften bei gleichzeitig hohem Tragekomfort und sicherer Handhabbarkeit bietet, sowie gleichzeitig auch eine ausgezeichnete Haltbarkeit und dadurch eine längere Lebensdauer aufweist.

Die Aufgabe der Erfindung wird durch ein Maskenkissen gemäß Anspruch 1 bzw. 12 bzw. eine nasale Beatmungsmaske gemäß Anspruch 22 bzw. 23 gelöst.

Bevorzugte Ausgestaltungen der Erfindung sind in den jeweiligen abhängigen Ansprüchen definiert.

Das Maskenkissen gemäß der Erfindung ist vorgesehen für eine nasale Beatmungsmaske und ist zur abdichtenden Verbindung mit dem Gesicht eines Benutzers an einem Maskenhalteteil anbringbar, wobei das Maskenhalteteil und das Maskenkissen einen Innenraum zur Aufnahme zumindest der Nase des Benutzers bilden. Erfindungsgemäß besteht das Maskenkissen aus einem flexiblen Material mit einer inneren Stützwand und mit einer nach innen um die Stützwand geschlagenen Dichtlippe. Die Dichtlippe verläuft auf der dem Innenraum abgewandten Seite der Stützwand und ist von dieser beabstandet. Die Dichtlippe liegt bei Gebrauch der Beatmungsmaske mit einem Teil ihrer äußeren Oberfläche auf dem Gesicht des Benutzers abdichtend auf und verformt sich dabei in Richtung der Stützwand. Erfindungsgemäß ist die Stützwand mit Stegen versehen, welche bei Gebrauch der Beatmungsmaske deren Verformung verringern.

Durch die erfindungsgemäße Ausgestaltung des Maskenkissens mit einer inneren Stützwand und einer nach innen um die Stützwand geschlagenen Dichtlippe, wobei die Stützwand mit Stegen versehen ist, welche die Stützwand versteifen und somit ihre Verformung bei Gebrauch der Beatmungsmaske verringern, wird gewährleistet, daß es zu einer sicher abdichtenden Verbindung des Maskenkissens mit dem Gesicht des Benutzers kommt, ohne daß das Maskenkissen mit übermäßig hoher Kraft gegen das Gesicht des Benutzers gedrückt werden müßte. Die Verformungsstabilität beugt der Entstehung von Falten vor, welche die Dichtwirkung des Maskenkissens verringern oder sogar aufheben könnten.

Besonders vorteilhaft ist es, wenn die Stützwand des erfindungsgemäßen Maskenkissens an ihrer nach innen gewandten Oberfläche, also auf ihrer dem Innenraum zugewandten Seite, und/oder an ihrer nach außen gewandten Oberfläche, d.h. an ihrer dem Innenraum abgewandten Seite, mit den entsprechenden Stegen versehen ist. Insbesondere bei Anordnung der Stege an der nach innen gewandten Oberfläche der Stützwand wird vermieden, daß sich die im wesentlichen auf der dem Innenraum abgewandten Seite der Stützwand verlaufende Dichtlippe zu eng an die ausgeformten Stege der Stützwand anlegt und eventuell abschnittsweise zwischen sie rutscht und somit wiederum die Gefahr einer Faltenbildung besteht. Durch Anordnung entsprechend ausgeformter Stege auf beiden Oberflächen der Stützwand wiederum ist eine besonders verformungsstabile Gestaltung des Maskenkissens zu erreichen.

In einer besonders vorteilhaften Ausführungsform der Erfindung verlaufen die Stege im wesentlichen senkrecht bezüglich einer umlaufenden Kante der Stützwand. Dabei ist diese Kante der umlaufende Abschluß der Stützwand. Durch die im wesentlichen senkrechte Anordnung der Stege kann auf einfache Weise die Verformung verringert werden, da üblicherweise die Kräfte, welche durch die Positionierung der nasalen Beatmungsmaske auftreten, im wesentlichen senkrecht zur Anlagefläche der Gesichtskontur und somit wiederum im wesentlichen senkrecht zur Abschlußkante der Stützwand verlaufen.

Insbesondere kann es jedoch auch vorteilhaft sein, die Stege bezüglich der umlaufenden Kante der Stützwand im wesentlichen schräg anzuordnen. Dies ist von Vorteil, da insbesondere in bestimmten Bereichen der Anlage des Maskenkissens an das Gesicht eines Benutzers die Richtung der beim Gebrauch auftretenden Verformungskräfte nicht zwangsläufig senkrecht zum Gesicht/der Gesichtskontur des Benutzers bzw. zur umlaufenden Kante der Stützwand verlaufen. Vielmehr können entsprechende Kräfte auch schräg auf das erfindungsgemäße Maskenkissen einwirken, insbesondere bei Schwenkbewegungen des Kopfes, so daß hier gezielt durch Ausrichtung der Stege in die entsprechenden Kraftrichtungen der Verformungskräfte eine besonders hohe Stabilität gegen Verformung gegeben ist, da diese Verformungskräfte so besonders gut aufgenommen werden können ohne zu entsprechenden Verformungen zu führen.

Selbstverständlich ist es auch möglich, über den Umfang des Maskenkissens verteilt die Stege senkrecht und/oder schräg anzuordnen, je nach Erfordernis entsprechend der Kontur des Gesichts eines Benutzers.

Um auch bei stärkerem Andrücken des erfindungsgemäßen Maskenkissens angenehme Trageeigenschaften zu gewährleisten, kann die umlaufende Kante der Stützwand nach innen umgeschlagen sein. Somit liegt sie im Extremfall nicht mit ihrer Kante in Richtung des Gesicht eines Benutzers an, sondern bildet vielmehr einen abgerundeten Bereich, welcher in Richtung des Gesichts eines Benutzers zeigt.

Bevorzugt ist es, daß die Dichtlippe des Maskenkissens im Verhältnis zur Stützwand flexibler gestaltet ist. Somit kann einerseits eine gute Abdichtung und andererseits eine hohe Verformungsbeständigkeit optimal gewährleistet werden.

Besonders vorteilhaft ist es, daß die Dichtlippe im Verhältnis zur Stützwand mit geringerer Materialstärke ausgeführt ist.

Um die Formstabilität und Haltbarkeit der äußerst flexiblen Dichtlippe ohne Einschränkung der guten Trageeigenschaften zu verbessern, ist es besonders vorteilhaft, falls die Dichtlippe des Maskenkissens in einer Ausführungsform der Erfindung eine mit einer Verdickung bzw. Wulst versehene umlaufende Kante ihres nach innen geschlagenen Bereichs aufweist. Diese verhindert das Einreißen der Dichtlippe in diesem Bereich.

Das flexible Maskenkissen ist an dem Maskenhalteteil anbringbar, wobei das Maskenhalteteil und das Maskenkissen einen Innenraum zur Aufnahme zumindest der Nase eines Benutzers bilden. Die Dichtlippe des Maskenkissens, welche bei Gebrauch der Beatmungsmaske mit einem Teil ihrer äußeren Oberfläche auf dem Gesicht des Benutzers abdichtend aufliegt, begrenzt gemäß der Erfindung in dem aufliegenden Teil mit einem Rand eine Öffnung zum Innenraum und der Rand weist eine Verdickung bzw. einen Wulst auf.

Durch die erfindungsgemäße Ausgestaltung des Maskenkissens mit einem Verdickung um die zur Aufnahme einer Nase dienende Öffnung, bewahrt das Maskenkissen sowohl seine Flexibilität und den Tragekomfort und ist trotzdem haltbar und reißfest. Durch die Verdickung kann der Rand der Öffnung darauf einwirkenden Kräften besser standhalten. Ein Einreißen des Maskenkissens ist damit weitestgehend ausgeschlossen. Das Maskenkissen kann von einem Benutzer über einen langen Zeitraum verwendet werden, was ihm unnötige Kosten für Ersatzkissen bzw. komplett neue Beatmungsmasken erspart. Ebenfalls bewirkt der so verdickte Randbereich eine bessere Abdichtwirkung, da er eine größere Formstabilität aufweist.

In einem bevorzugten Ausführungsbeispiel des Maskenkissens ist die Verdickung umlaufend um die gesamte Öffnung ausgebildet. Diese umlaufende Ausbildung der Verdickung gewährt größtmögliche Haltbarkeit des Maskenkissens und vereinfacht auch die Herstellung desselben. Vorzugsweise besteht das Maskenkissen aus einem flexiblen, elastischem Material. Besonders geeignet ist Silikon, aber es ist ebenfalls möglich, andere flexible und hautverträgliche Materialien zu verwenden.

Die Verbindung des Maskenkissens mit dem Maskenteil erfolgt in einer Ausführungsform der Erfindung vorzugsweise durch Einrasten eines angeformten umlaufenden Wulstes des Maskenkissens in einer umlaufenden nutenförmigen Aussparung des Maskenteils.

Ergonomisch ist es besonders bevorzugt, daß das Maskenkissen gemäß einer weiteren Ausführungsform der Erfindung eine im wesentlichen dreieckige Grundform aufweist und besonders bevorzugt die innere Stützwand und die Dichtlippe des Maskenkissens bzw. das Maskenkissen an sich eine umlaufende Kontur aufweisen, welche im wesentlichen der allgemeinen Gesichtskontur eines Benutzers entspricht. Unter allgemeiner Gesichtskontur eines Benutzers ist dabei die Gesichtskontur zu verstehen, welche sich beispielsweise bei Aufsetzen einer nasalen Beatmungsmaske im Bereich um die Nase eines Benutzers darstellt.

Vorzugsweise besteht das erfindungsgemäße Maskenkissen aus Silikon, es ist jedoch auch möglich, andere flexible und hautverträgliche Materialien zu verwenden.

Eine erfindungsgemäße nasale Beatmungsmaske weist im wesentlichen ein Maskenkissen und ein Maskenhalteteil auf, wobei das Maskenhalteteil mit einem Schlauchanschluß für einen Beatmungsschlauch in Verbindung steht. Ferner weist die nasale Beatmungsmaske mindestens ein Gurtband auf, welches zur Positionierung der Beatmungsmaske auf dem Gesicht, insbesondere auf der Nase eines Benutzers, dient. Das Maskenkissen und das Maskenhalteteil sind dabei entsprechend den Merkmalen des Anspruchs 1 und/oder 12 vorgesehen. Die bevorzugten Gestaltungen des Maskenkissens, welche sich aus den entsprechenden Unteransprüchen und der Beschreibung ergeben, sollen auch im Zusammenhang mit der erfindungsgemäßen nasalen Beatmungsmaske gegeben sein.

Die besonders einfache Handhabbarkeit der erfindungsgemäßen nasalen Beatmungsmaske ist in einer bevorzugten Ausführungsform dann gegeben, wenn das Gurtband der nasalen Beatmungsmaske einen Schnellverschluß zur lösbaren Positionierung der Beatmungsmaske aufweist. Dies erlaubt eine besonders einfache Handhabung, wobei aufgrund der erfindungsgemäßen Gestaltung, insbesondere auch des Maskenkissens, durch die schnell vorzunehmende Positionierung der Beatmungsmaske keine Einschränkung der Dichtheit oder des Tragekomforts der nasalen Beatmungsmaske aufgrund von dadurch eventuell auftretenden Verformungen, welche durch den Schnellverschluß und den dadurch besonders raschen An- und Ablegeprozeß der nasalen Beatmungsmaske gegeben sind, auftreten können.

Die Erfindung wird im folgenden anhand der beigefügten Zeichnung näher erläutert. Dabei zeigt:
- Fig. 1: eine teilweise freigeschnittene perspektivische Ansicht eines Maskenkissens für eine nasale Beatmungsmaske gemäß einer ersten bevorzugten Ausführungsform der Erfindung.
- Fig. 2: eine teilweise freigeschnittene perspektivische Ansicht eines Maskenkissens für eine nasale Beatmungsmaske gemäß einer zweiten bevorzugten Ausführungsform der Erfindung.
- Fig. 3: einen vergrößerten Ausschnitt der Verdickung des erfindungsgemäßen Maskenkissens gemäß Fig. 1 und 2.
- Fig. 4: eine schematische Frontansicht einer nasalen Beatmungsmaske.

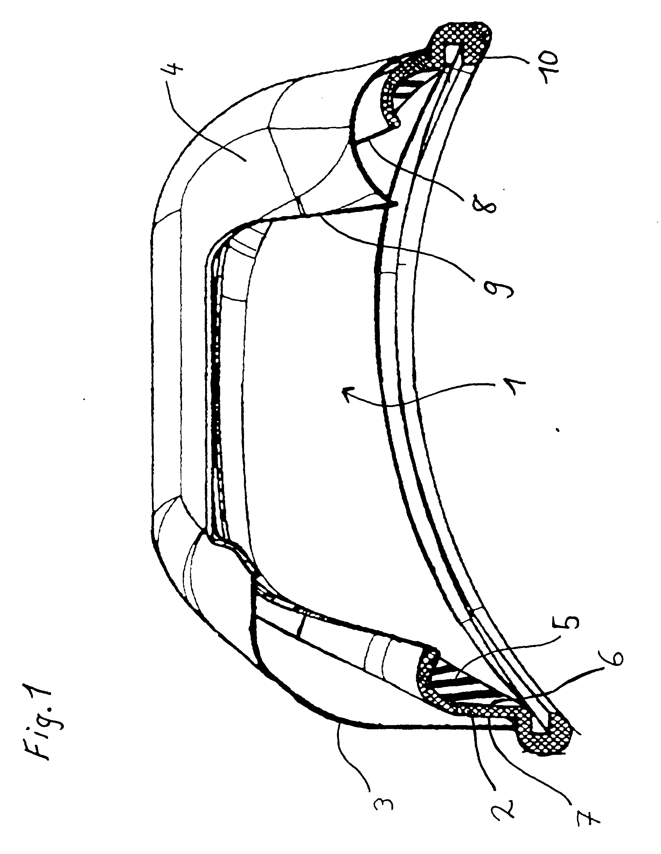

Fig. 1 zeigt das erfindungsgemäße Maskenkissen gemäß einer ersten bevorzugten Ausführungsform der Erfindung ohne die Abbildung der dazugehörigen entsprechenden nasalen Beatmungsmaske.

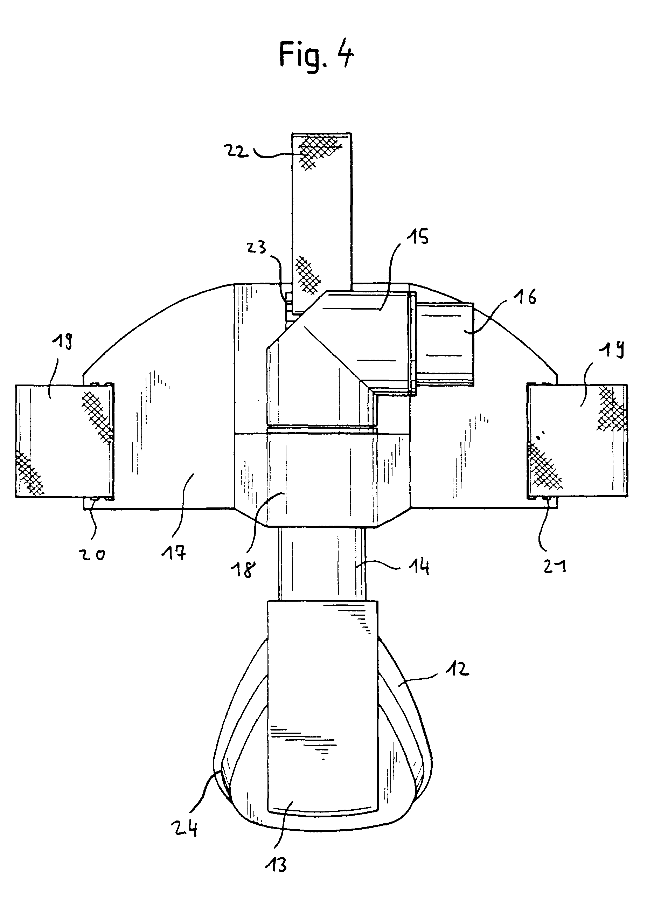

Das dargestellte Maskenkissen ist zur abdichtenden Verbindung mit dem Gesicht eines Benutzers an einem Maskenhalteteil (nicht gezeigt) anbringbar, wobei Maskenteil und Maskenkissen einen Innenraum 1 zur Aufnahme zumindest der Nase des Benutzers bilden. Das Maskenkissen besteht aus flexiblem Material und weist eine innere Stützwand 2 auf, mit einer nach innen gewandten Oberfläche 6 und einer nach außen gewandten Oberfläche 7. Eine Dichtlippe 3 ist nach innen um die Stützwand 2 geschlagen, wobei die Dichtlippe 3, wie in Fig. 1 gezeigt, auf der dem Innenraum abgewandten Seite, d.h. auf der Seite der nach außen gewandten Oberfläche 7 der Stützwand 2, verläuft. Ferner ist die Dichtlippe 3 von der Stützwand 2 beabstandet. Die Dichtlippe 3 weist einen Teil 4 ihrer äußeren Oberfläche auf, welcher bei Gebrauch der Beatmungsmaske auf dem Gesicht des Benutzers abdichtend aufliegt. Dabei verformt sich die Dichtlippe 3 in Richtung der Stützwand 2. Ferner weist die Dichtlippe 3 eine mit einer Verdickung 11 versehene umlaufende Kante 9 ihres nach innen geschlagenen Teils 4 auf, welche im Detail in Fig. 3 vergrößert dargestellt ist. Auch die Kante 8 der Stützwand 2 ist im Fall der gezeigten Ausführungsform nach innen in Richtung des Innenraums 1 umgeschlagen. Das Maskenkissen ist durch Einrasten eines angeformten umlaufenden Wulstes 10 in einer entsprechenden umlaufenden nutenförmigen Aussparung des Maskenteils mit diesem verbindbar.

Erfindungsgemäß sind Stege 5 vorgesehen, welche bei Gebrauch die Verformung der Stützwand 2 verringern. Im Fall der gezeigten Ausführungsform sind die Stege 5 an der nach innen gewandten Oberfläche 6 der Stützwand 2, d.h. an der dem Innenraum 1 zugesandten Seite der Stützwand 2, und im wesentlichen senkrecht bezüglich der Kante 8 der Stützwand 2 angeordnet. Die vermeintliche Schrägstellung der Stege ergibt sich aus der perspektivischen Darstellung gemäß der Fig. 1.

Das Maskenkissen der gezeigten Ausführungsform der Erfindung ist von im wesentlichen dreieckiger Grundform, wobei aufgrund der Perspektive der Fig. 1 und aufgrund des Schnitts durch das Maskenkissen diese Grundform schlecht zu erkennen ist.

Die gesamte nasale Beatmungsmaske ist in Fig. 1 nicht dargestellt. Die erfindungsgemäße nasale Beatmungsmaske weist in ihrer bevorzugten Ausführungsform jedoch ein Maskenkissen auf, welches dem in Fig. 1 dargestellten Maskenkissen entspricht.

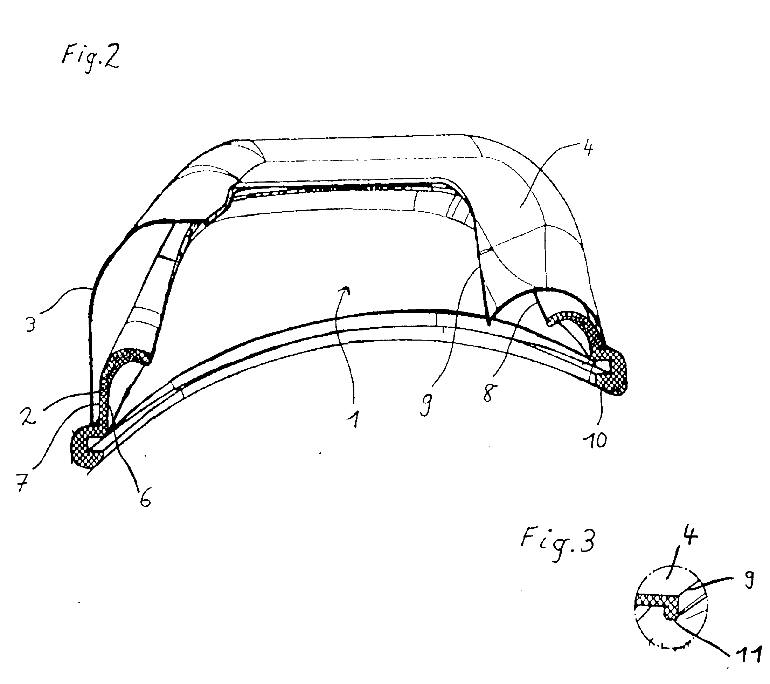

Fig. 2 zeigt eine zweite Ausführungsform des erfindungsgemäßen Maskenkissens. Das flexible Maskenkissen und das Maskenhalteteil (nicht gezeigt) bilden einen Innenraum 1, welcher zur Aufnahme der Nase eines Benutzers dient. Das Maskenkissen weist eine innere Stützwand 2 mit einer nach innen gewandten Oberfläche 6 auf. Eine Dichtlippe 3 ist nach innen um die Stützwand 2 geschlagen, wobei die Dichtlippe 3 auf der dem Innenraum 1 abgewandten Seite der Stützwand 2 verläuft. Zwischen der Dichtlippe 3 und der Stützwand 2 befindet sich - wie hier dargestellt im unbelasteten Zustand - ein schmaler Zwischenraum. Die Dichtlippe 3 weist einen Teil 4 an ihrer äußeren Oberfläche auf, welcher bei Gebrauch der Beatmungsmaske auf dem Gesicht des Benutzers abdichtend aufliegt. Darüber hinaus weist die Dichtlippe 3 in dem aufliegenden Teil 4 eine Kante bzw. einen Rand 9 auf, welche(r) eine Öffnung zum Innenraum 1 begrenzt. Die Kante 9 ist mit einem um die Öffnung umlaufenden Verdickung 11 versehen, welche im Detail in Fig. 3 vergrößert dargestellt ist. Das Maskenkissen weist einen angeformten umlaufenden Wulst 10 auf, welcher zur Befestigung mit dem Maskenhalteteil in eine nutenförmige Aussparung davon eingreift (nicht gezeigt).

Fig. 3 zeigt einen vergrößerten Ausschnitt der Fig. 1 und 2, in welchem die um die Öffnung der Dichtlippe 3 laufende Verdickung 11 dargestellt ist, wobei die Materialdicke der Dichtlippe 3 im Randbereich bzw. im Bereich der Kante 9 etwa verdoppelt ist.

Fig. 4 zeigt eine schematische Frontansicht einer nasalen Beatmungsmaske.

Die in Figur 4 gezeigte Ausführungsform einer nasalen Beatmungsmaske besteht aus einem flexiblen Maskenteil 12 gemäß der Erfindung zur Anpassung der Beatmungsmaske an die Anatomie des Patienten. Das flexible Maskenteil 12 ist lösbar mit einem Maskenhalteteil 13 verbunden.

An das Maskenhalteteil 13 ist ein längliches Rohr 14 angesetzt, welches vertikal angeordnet ist.

An dem oberen Ende des Rohres 14 befindet sich ein Winkelrohr 15. Das als Knierohr ausgebildete Winkelrohr 15 ist gegen das längliche Rohr 14 radial verdrehbar ausgeführt. Am freien Ende des horizontalen Teiles des Winkelrohrs 15 befindet sich ein Schlauchanschluß 16 für einen nicht gezeigten Beatmungsschlauch. Dieser Schlauchanschluß 16 ist zur zusätzlichen Steigerung der Bewegungsfreiheit axial um den horizontalen Teil des Winkelrohres 15 drehbar.

Ein Schild 17 ist über die Stirnschildaufnahme 18 zwischen Anschlägen des Maskenhalteteiles 13 und des vertikalen Teiles des Winkelrohrs 15 auf dem länglichen Rohr 14 verschiebbar gelagert.

An dem Stirnschild 17 ist ein Gurtband 19 mittels Ösen 20 und 21 in seiner Länge verstellbar befestigt. Zusätzlich zu dem Gurtband 19 ist ein Gurtband 22 ebenfalls in einer Öse 23 an der Oberseite des Stirnschildes 17 befestigt, welches zur stabileren Halterung der nasalen Beatmungsmaske über den Kopf geführt und am Hinterkopf mit dem Gurtband 19 verbunden ist. Die Gurtbänder bestehen im gezeigten Ausführungsbeispiel aus einem elastischen Material.

Die Positionierung der nasalen Beatmungsmaske erfolgt durch Überziehen der Einheit über den Kopf des Benutzers. Dabei wird der sich aus der Anatomie des Benutzers ergebende optimale Abstand zwischen dem Stirnschild 17 mit dem Gurtband 19 und dem Maskenteil 12 durch das Verschieben der Stirnschildaufnahme 18 auf dem länglichen Rohr 14 eingestellt. Außerdem erfolgt durch das Verstellen des Gurtbandes 19 und des zusätzlichen Gurtbandes 22 mittels der Ösen 20, 21, 23 eine Anpassung der Halterung der Beatmungsmaske an die Kopfform des Benutzers sowie durch unterschiedlich starkes Festziehen der Gurtbänder auch die gewünschte Fixierung der Beatmungsmaske. Über den Schlauchanschluß 16 erfolgt der Anschluß des Beatmungsschlauches.

Die Verbindung des Maskenteils 12 mit dem Maskenhalteteil 13 erfolgt derart, daß in dem aus elastischem Material bestehenden Maskenteil 12 eine ringförmige Wulst 10 ausgeformt ist, welche in eine ringförmige Aussparung 24 des Maskenhalteteils eingedrückt ist. Somit sind das Maskenteil 12 und das Maskenhalteteil 13 in der gezeigten Weise mechanisch stabil und luftdicht miteinander verbunden.

## Patentansprüche

1. Maskenkissen für eine nasale Beatmungsmaske, welches zur abdichtenden Verbindung mit einem Gesicht eines Benutzers an einem Maskenhalteteil anbringbar ist, wobei Maskenhalteteil und Maskenkissen einen Innenraum (1) zur Aufnahme zumindest der Nase des Benutzers bilden, aus flexiblem Material, mit einer inneren Stützwand (2) und einer und nach innen um die Stützwand (2) geschlagenen Dichtlippe (3), wobei die Dichtlippe (3) auf der dem Innenraum (1) abgewandten Seite der Stützwand (2) verläuft und von dieser beabstandet ist, wobei die Dichtlippe (3) bei Gebrauch der Beatmungsmaske mit einem Teil (4) ihrer äußeren Oberfläche auf dem Gesicht des Benutzers abdichtend aufliegt und sich dabei in Richtung der Stützwand (2) verformt und die Stützwand (2) mit Stegen (5) versehen ist, welche bei Gebrauch der Beatmungsmaske deren Verformung verringern.

2. Maskenkissen für eine nasale Beatmungsmaske nach Anspruch 1, dadurch gekennzeichnet, daß die Stützwand (2) an ihrer nach innen gewandten Oberfläche (6) und/oder an ihrer nach außen gewandten Oberfläche (7) mit Stegen (5) versehen ist.

3. Maskenkissen nach Anspruch 1 oder 2, dadurch gekennzeichnet, daß die Stege (5) im wesentlichen senkrecht bezüglich einer umlaufenden Kante (8) der Stützwand (2) verlaufen.

4. Maskenkissen nach Anspruch 1 oder 2, dadurch gekennzeichnet, daß die Stege (5) im wesentlichen schräg bezüglich einer umlaufenden Kante (8) der Stützwand (2) verlaufen, insbesondere in Richtung von beim Gebrauch auftretenden Verformungskräften.

5. Maskenkissen nach Anspruch 3 oder 4, dadurch gekennzeichnet, daß die umlaufende Kante (8) der Stützwand (2) nach innen umgeschlagen ist.

6. Maskenkissen nach einem der Ansprüche 1 bis 5, dadurch gekennzeichnet, daß die Dichtlippe (3) im Verhältnis zur Stützwand (2) flexibler ist.

7. Maskenkissen nach einem der Ansprüche 1 bis 6, dadurch gekennzeichnet, daß die Dichtlippe (3) im Verhältnis zur Stützwand (2) mit geringerer Materialstärke ausgeführt ist.

8. Maskenkissen nach einem der Ansprüche 1 bis 7, dadurch gekennzeichnet, daß die Dichtlippe (3) eine mit einer Verdickung (11) versehene umlaufende Kante (9) ihres nach innen geschlagenen Teils aufweist.

9. Maskenkissen nach einem der Ansprüche 1 bis 8, dadurch gekennzeichnet, daß es durch Einrasten eines angeformten umlaufenden Wulstes (10) in einer umlaufenden nutenförmigen Aussparung des Maskenteils mit diesem verbindbar ist.

10. Maskenkissen nach einem der Ansprüche 1 bis 9, dadurch gekennzeichnet, daß es eine im wesentlichen dreieckige Grundform aufweist und die innere Stützwand (2) und die Dichtlippe (3) eine umlaufende Kontur aufweisen, welche im wesentlichen der allgemeinen Gesichtskontur eines Benutzers entspricht.

11. Maskenkissen nach einem der Ansprüche 1 bis 10, dadurch gekennzeichnet, daß es aus Silikon besteht.

12. Maskenkissen für eine nasale Beatmungsmaske, welches flexibel ist und an einem Maskenhalteteil anbringbar ist, wobei Maskenhalteteil und Maskenkissen einen Innenraum (1) zur Aufnahme zumindest der Nase eines Benutzers bilden, mit einer Dichtlippe (3), welche bei Gebrauch der Beatmungsmaske mit einem Teil (4) ihrer äußeren Oberfläche auf dem Gesicht des Benutzers abdichtend aufliegt, wobei die Dichtlippe (3) in dem aufliegenden Teil (4) mit einer Kante (9) eine Öffnung zum Innenraum (1) begrenzt und die Kante (9) eine Verdickung (11) aufweist.

13. Maskenkissen nach Anspruch 12, bei welchem die Kante (9) der Öffnung als umlaufender Verdickung (11) ausgebildet ist.

14. Maskenkissen nach Anspruch 12 oder 13, welches aus flexiblem Material, insbesondere aus Silikon, besteht.

15. Maskenkissen nach einem der Ansprüche 12 bis 14, welches einen angeformten Wulst (10) aufweist, welcher zur Befestigung mit dem Maskenhalteteil in eine nutenförmigen Aussparung davon eingreift.

16. Maskenkissen nach einem der Ansprüche 12 bis 15, welches eine innere Stützwand aufweist, wobei die Dichtlippe (3) nach innen um die Stützwand geschlagen ist.

17. Maskenkissen nach Anspruch 16, bei welchem die Dichtlippe (3) im Verhältnis zur Stützwand mit geringerer Materialstärke ausgebildet ist.

18. Maskenkissen nach Anspruch 16 oder 17, bei welchem die Dichtlippe (3) im Verhältnis zur Stützwand flexibler ist.

19. Maskenkissen nach einem der Ansprüche 16 bis 18, bei welchem die Stützwand mit Stegen versehen ist, welche bei Gebrauch der Beatmungsmaske deren Verformung verringern.

20. Maskenkissen nach Anspruch 19, bei welchem die Stützwand an ihrer nach innen gewandten Oberfläche mit Stegen versehen ist, wobei die Stege im wesentlichen senkrecht bezüglich einer umlaufenden Kante der Stützwand verlaufen.

21. Maskenkissen nach Anspruch 19, bei welchem die Stege im wesentlichen schräg bezüglich der umlaufenden Kante der Stützwand verlaufen.

22. Nasale Beatmungsmaske mit einem Maskenkissen und einem Maskenhalteteil, das mit einem Schlauchanschluß für einen Beatmungsschlauch in Verbindung steht, und mit mindestens ein Gurtband zur Positionierung der Beatmungsmaske auf dem Gesicht, insbesondere auf der Nase eines Benutzers, wobei Maskenhalteteil und Maskenkissen einen Innenraum (1) zur Aufnahme zumindest der Nase des Benutzers bilden, und wobei das Maskenkissen aus flexiblem Material besteht, mit einer inneren Stützwand (2) und einer und nach innen um die Stützwand (2) geschlagenen Dichtlippe (3), wobei die Dichtlippe (3) auf der dem Innenraum (1) abgewandten Seite der Stützwand (2) verläuft und von dieser beabstandet ist, wobei die Dichtlippe (3) bei Gebrauch der Beatmungsmaske mit einem Teil (4) ihrer äußeren Oberfläche auf dem Gesicht des Benutzers abdichtend aufliegt und sich dabei in Richtung der Stützwand (2) verformt und die Stützwand (2) mit Stegen (5) versehen ist, welche bei Gebrauch der Beatmungsmaske deren Verformung durch die auftretenden Verformungskräfte, welche durch die Positionierung durch das Gurtband auftreten, verringern.

23. Nasale Beatmungsmaske mit einem Maskenkissen, insbesondere mit den Merkmalen gemäß einem der Ansprüche 12 bis 21, und einem Maskenhalteteil, das mit einem Schlauchanschluß für einen Beatmungsschlauch in Verbindung steht und mindestens ein Gurtband zur Positionierung der Beatmungsmaske auf dem Gesicht, insbesondere auf der Nase eines Benutzers, aufweist, wobei Maskenhalteteil und Maskenkissen einen Innenraum (1) zur Aufnahme zumindest der Nase eines Benutzers bilden, wobei das Maskenkissen eine Dichtlippe (3) aufweist, welche bei Gebrauch der Beatmungsmaske mit einem Teil (4) ihrer äußeren Oberfläche auf dem Gesicht des Benutzers abdichtend aufliegt, wobei die Dichtlippe (3) in dem aufliegenden Teil (4) mit einer Kante (9) eine Öffnung zum Innenraum (1) begrenzt und die Kante eine Verdickung (11) aufweist.

24. Nasale Beatmungsmaske nach Anspruch 22 oder 23, bei welcher das Gurtband einen Schnellverschluß zur lösbaren Positionierung der Beatmungsmaske aufweist.
